# EUROPEAN PATENT APPLICATION

(11) **EP 4 249 077 A1**
(43) Date of publication of application: **27.09.2023**
(21) Application number: 21894629.1
(22) Date of filing: 16.11.2021
(51) Int. Cl.: A61P 43/00, B01J 35/02, C07B 53/00, C07D 219/02, C07D 219/04, C07D 219/06, C07D 219/08, C07F 5/02, C07B 61/00, C07D 498/14, B01J 31/02, A61K 31/5383

(54) **NOVEL ACRIDINIUM SALT AND METHOD FOR PRODUCING SAME**

(30) Priority: 17.11.2020 JP 2020191118
(71) Applicant: Shionogi & Co., Ltd, Osaka-shi, Osaka 541-0045 (JP); Shionogi Pharma Co., Ltd., Settsu-shi, Osaka 566-0022 (JP)
(72) Inventor: MAJIMA, Shohei, Amagasaki-shi, Hyogo 660-0813 (JP)
(74) Representative: Mewburn Ellis LLP
(86) International application number: PCT/JP2021/042049
(87) International publication number: WO 2022/107755

(57) **Abstract**

The present invention provides novel aclidinium salt of the formula (I) and a process for the production of the aclidinium salt. 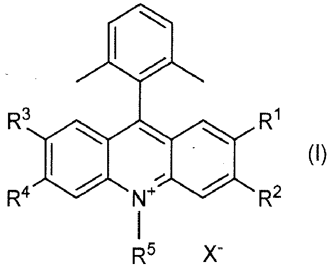 wherein R¹ is C1-C6 alkyl or C1-C6 alkyloxy; R² is hydrogen or C1-C6 alkyloxy; R³ is hydrogen, halogen, C1-C6 alkyl or C1-C6 alkyloxy; R⁴ is hydrogen, C1-C6 alkyloxy, halo C1-C6 alkyloxy or C1-C6 alkylamino; R⁵ is C1-C3 alkyl; and X⁻ is an anion.

## Description

### [Technical field]

The present invention relates to a novel acridinium salt useful as a photocatalyst and a method for producing the same.

### [Background Art]

Tricyclic pyridone derivatives, such as 7-Hydroxy-3,4,12,12a-tetrahydro-1H-[1,4]oxadino[3,4-c]pyrido[2,1-f] [1,2,4]triazine-6,8-dione, are known as a parent skeleton of substituted polycyclic pyridone derivatives having a cap-dependent endonuclease inhibitory activity and also known as a skeleton common to other compounds useful as pharmaceuticals, such as those having HIV integrase inhibitory activity. Industrial friendly processes for synthesizing skeletal moieties common to such pharmaceutically useful compounds are being development.

Patent Documents 1 to 14 describe a process, as shown below, for the stereoselective production of dolutegravir using (R)-aminoalcohol. 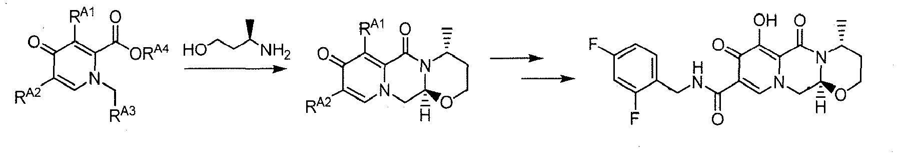 wherein R^{A1} is hydroxy, alkyloxy, halogen or the like; R^{A2} is hydrogen, difluorobenzylcarbamoyl, alkyloxycarbonyl, carboxy or the like; R^{A3} is aldehyde or aldehyde equivalent; R^{A4} is alkyl or the like.

Patent Documents 15 and 16 describe a process, as shown below, wherein the enantiomeric mixture of 7-(benzyloxy)-3,4,12,12a-tetrahydro-1H-[1,4]oxadino[3,4-c]pyrido[2,1-f][1,2,4]triazine-6,8-dione is synthesized and then optically resolved to obtain an optically active substance. However, the process is not efficient because the compound having desired configuration is obtained once the enantiomeric mixture (1:1) is synthesized. 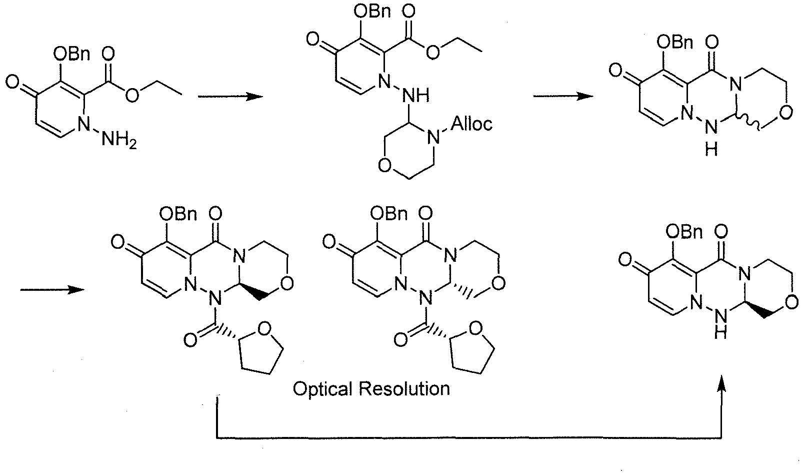 Patent Document 17 describes a process, as shown below, wherein an optically active ester group on the carbon adjacent to the amide is involves in a stereo selective cyclization, and the ester group is then hydrolyzed into a carboxy group, which is removed under light irradiation in the presence of an acridinium salt. However, this process is problematic for commercial purpose because the elimination reaction time is as long as 14 hours.

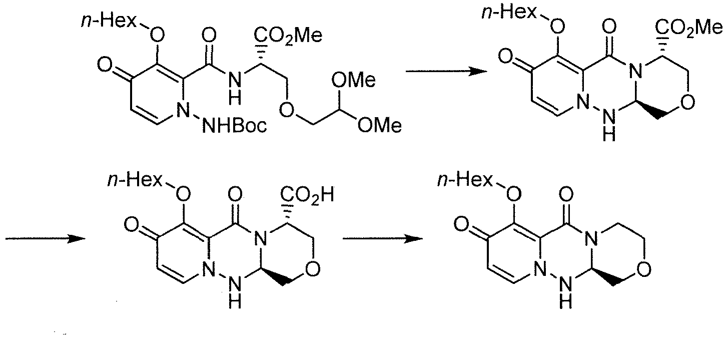

### [Prior Art Documents]

### [Patent Documents]

[Patent Document 1] WO 2010/011812
[Patent Document 2] WO 2010/011819
[Patent Document 3] WO 2010/068253
[Patent Document 4] WO 2010/068262
[Patent Document 5] WO 2010/110409
[Patent Document 6] WO 2012/018065
[Patent Document 7] WO 2014/128545
[Patent Document 8] WO 2015/009927
[Patent Document 9] WO 2015/019310
[Patent Document 10] WO 2015/110897
[Patent Document 11] WO 2015/111080
[Patent Document 12] WO 2015/177537
[Patent Document 13] WO 2016/092527
[Patent Document 14] WO 2016/125192
[Patent Document 15] WO 2016/175224
[Patent Document 16] WO 2017/104691
[Patent Document 17] WO 2019/070059

### Summary of Invention

An object of the present invention is to provide with a process wherein a removable functional group, such as carboxy group, is removed in a short time under light irradiation to obtain a product in high yield. Specifically, the present invention provides a catalyst for efficient production of intermediates, which are important in the process for the production of substituted polycyclic pyridone derivatives, such as a compound of the formula (VIIIa) or the formula (IXa) or a salt thereof as described herein. The present invention also provides a process for the efficient production of the catalyst.

The present inventors have found a catalyst for efficient production of an intermediate, which is important in the process for the production of a compound of the formula (VIIIa) or the formula (IXa), and also found a process for the production of the catalyst. That is, the inventors have found a catalyst to obtain optically-active substituted tricyclic pyridone derivatives of the formula (VII) with high yield and high enantioselectivity in a short time, in a process wherein a compound of the formula (IV) having a removable functional group (e.g., carboxy group) on the asymmetric carbon is prepared and then the removable functional group is removed. The inventor also have found reaction conditions for removing carboxy group under light irradiation in a short time to obtain a product in good yield. The inventors also have found an efficient process for the production of an acridinium salt of the formula (I).

The present invention provides the followings.
(1) An aclidinium salt of the formula (I): 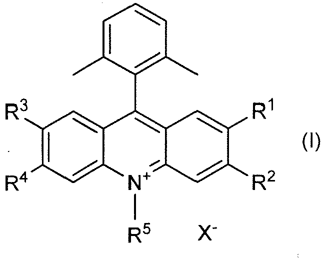 wherein
   R¹ is C1-C6 alkyl or C1-C6 alkyloxy;
   R² is hydrogen or C1-C6 alkyloxy;
   R³ is hydrogen, halogen, C1-C6 alkyl or C1-C6 alkyloxy;
   R⁴ is hydrogen, C1-C6 alkyloxy, halo C1-C6 alkyloxy or C1-C6 alkylamino;
   R⁵ is C1-C3 alkyl; and
   X⁻ is an anion.
(2) The acridinium salt according to (1), wherein R¹ is C1-C3 alkyloxy.
(3) The acridinium salt according to (1) or (2), wherein R² is hydrogen or C1-C3 alkyloxy.
(4) The acridinium salt according to any one of (1) to (3), wherein R³ is hydrogen, halogen, C1-C3 alkyl or C1-C3 alkyloxy.
(5) The acridinium salt according to any one of (1) to (4), wherein R⁴ is C1-C3 alkyloxy, halo C1-C3 alkyloxy or dimethylamino.
(6) The acridinium salt according to any one of (1) to (5), wherein R⁵ is methyl.
(7) The acridinium salt according to any one of (1) to (6), wherein R¹ is C1-C3 alkyloxy and R⁴ is C1-C3 alkyloxy.
(8) The acridinium salt according to (1) selected from the followings: 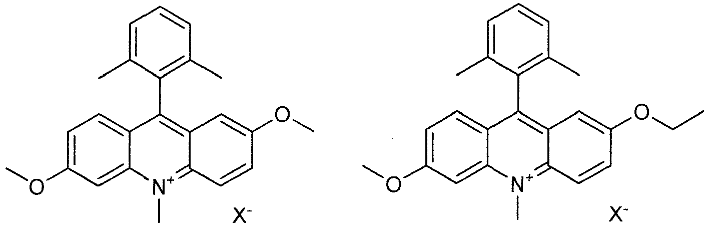 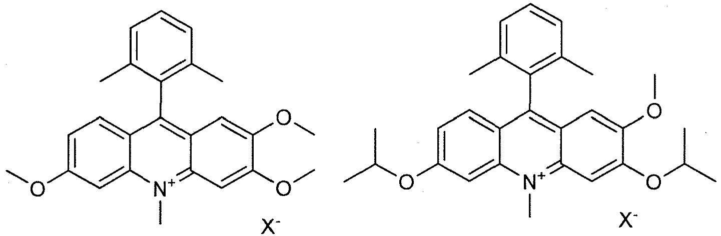 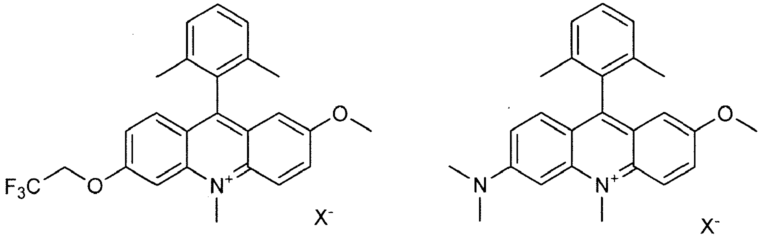 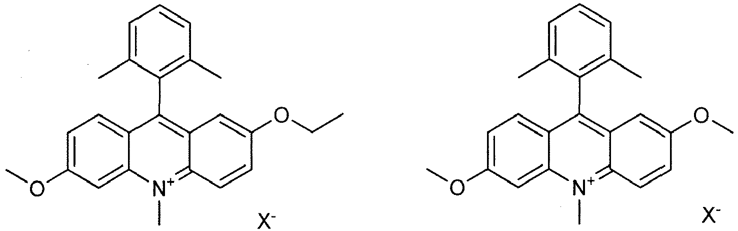 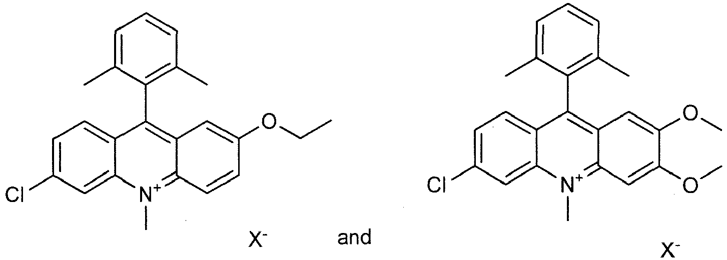 wherein X⁻ is as defined above in (1).
(9) The acridinium salt according to any one of (1) to (8), wherein X⁻ is BF₄⁻ or ClO₄⁻.
(10) A process for the production of a compound of the formula (VII): 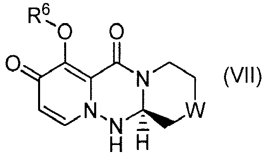
   wherein W is O or CH₂ and R⁶ is hydrogen or a hydroxyl protecting group,
   characterized by removing RA from a compound of the formula (IV) : 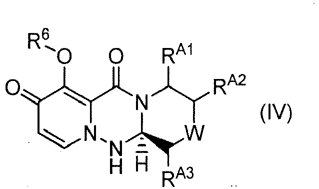 wherein
      R⁶ is hydrogen or a hydroxyl protecting group;
      R^{A1} is hydrogen or RA;
      R^{A2} is hydrogen or RA;
      R^{A3} is hydrogen or RA;
      W is O, CH₂ or CHRA;
      RA is a removable functional group;
      the carbon atom to which the RA is bonded is optically active; and
      provided that one of R^{A1}, R^{A2} and R^{A3} is RA and the other two are hydrogen and W is O or CH₂; or R^{A1}, R^{A2} and R^{A3} are hydrogen and W is CHRA,
   in the presence of the acridinium salt according to any one of (1) to (9).
(11) A process for the production of a compound of the formula (VII): 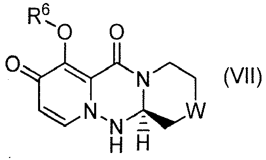
   wherein W is O or CH₂ and R⁶ is hydrogen or a hydroxyl protecting group,
   characterized by removing RA from a compound of the formula (IV): 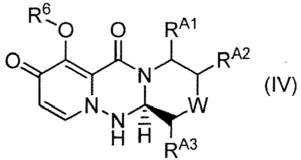 wherein
      R⁶ is hydrogen or a hydroxyl protecting group;
      R^{A1} is hydrogen or RA;
      R^{A2} is hydrogen or RA;
      R^{A3} is hydrogen or RA;
      W is O, CH₂ or CHRA;
      RA is a removable functional group;
      the carbon atom to which the RA is bonded is optically active; and
      provided that one of R^{A1}, R^{A2} and R^{A3} is RA and the other two are hydrogen and W is O or CH₂; or R^{A1}, R^{A2} and R^{A3} are hydrogen and W is CHRA,
   in the presence of an acridinium salt, imidazole and 4-isopropylbenzenethiol, under light irradiation in one or more solvents selected from the group consisting of ethanol and dichloromethane.
(12) The process according to (11), wherein RA is removed in the presence of the aclidinium salt according to any one of (1) to (9).
(13) A process for the production of a compound of the formula (VIIIa) or formula (IXa) or a salt thereof: 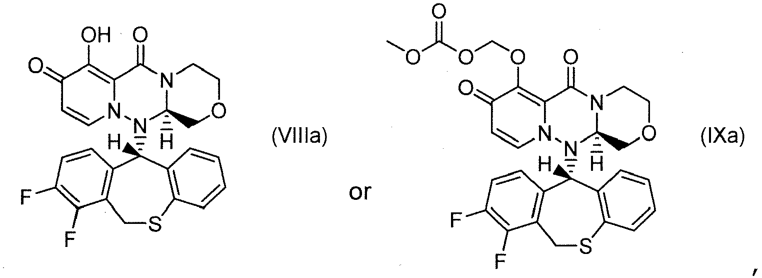 comprising the process according to any one of (10) to (12).
(14) A process for the production of an aclidinium salt of the formula (III): 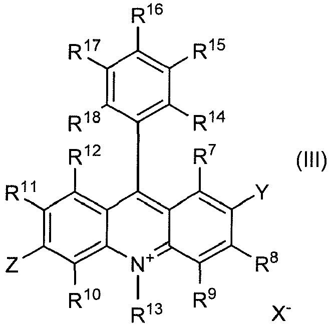 wherein
   R⁷ to R¹² are independently hydrogen, C1-C6 alkyloxy, halo C1-C6 alkyloxy or C1-C6 alkylamino;
   R¹³ is a substituted or unsubstituted C1-C6 alkyl or aromatic carbocyclyl;
   R¹⁴ to R¹⁸ are independently hydrogen, halogen, C1-C6 alkyloxy, halo C1-C6 alkyloxy or C1-C6 alkylamino;
   Y is C1-C6 alkyloxy;
   Z is a leaving group; and
   X⁻ is an anion,
   characterized in that a compound of the formula (II) or a salt thereof: 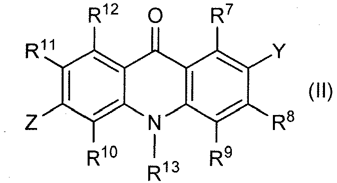 wherein
   R⁷ to R¹² are independently hydrogen, C1-C6 alkyloxy, halo C1-C6 alkyloxy or C1-C6 alkylamino;
   R¹³ is a substituted or unsubstituted C1-C6 alkyl or aromatic carbocyclyl;
   Y is C1-C6 alkyloxy; and
   Z is a leaving group,
   is reacted with a monocyclic aryl metal reagent and then treated with an acid.
(15) The process according to (14), wherein the monocyclic aryl metal reagent is a monocyclic aryl Grignard reagent.
(16) The process according to (14) or (15), wherein the reaction is carried out in the presence of a lanthanum chloride/lithium dichloride complex.
(17) A process for the production of an acridinium salt of the formula (IV): 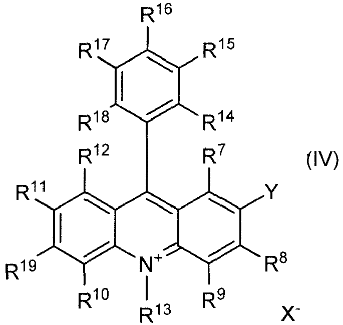 wherein
   R⁷ to R¹² are independently hydrogen, C1-C6 alkyloxy, halo C1-C6 alkyloxy or C1-C6 alkylamino;
   R¹³ is a substituted or unsubstituted C1-C6 alkyl or aromatic carbocyclyl;
   R¹⁴ to R¹⁸ are independently hydrogen, halogen, C1-C6 alkyloxy, halo C1-C6 alkyloxy or C1-C6 alkylamino;
   R¹⁹ is C1-C6 alkyloxy;
   Y is C1-C6 alkyloxy; and
   X⁻ is an anion,
   characterized in that an acridinium salt of the formula (III): 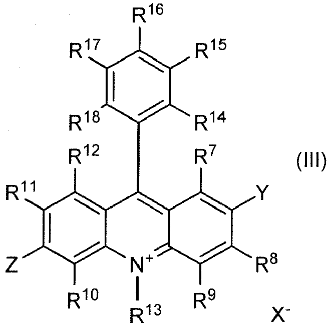
   wherein Z is a leaving group, and the other symbols are as defined above,
   is reacted with a nucleophile.
(18) The process according to (17) wherein the nucleophile is a metal C1-C6 alkoxide or C1-C6 alkylamine.
(19) The process according to (17) or (18) wherein the aclidinium salt of the formula (III) is that obtained by the process according to (14) or (15).

### Effect of the Invention

By the present invention, a removable functional groups, such as carboxy group, is removed efficiently under light irradiation in a short time. Furthermore, the acridinium salt of the invention can be used in a reaction for removing any removable functional group, which is not limited to carboxy group. Further, the acridinium salt of the invention can be used not only for elimination reactions but also for any reaction that proceeds in the presence of the acridinium salt.

In addition, the present invention is able to prepare efficiently 2,6-dialkyloxyacrydinium salt, which is difficult to be synthesized.

### Description of Embodiments

The meaning of each term used in the present description is explained below. Unless otherwise specified, each term has the same meaning when used alone or in combination with another term.

The term "consisting of" or "consists of" means that the named element(s) is included exclusively.

The terms "including", "include", "comprising" and "comprises" mean to include, but not limited to, the named element(s), i.e., other element(s) is not excluded.

The term "halogen" includes fluorine, chlorine, bromine and iodine. Fluorine and chlorine are preferable, and fluorine is especially preferable.

The term "C1-C6 alkyl" means a linear or branched alkyl having 1 to 6 carbon atoms, and includes an alkyl having 1 to 4 carbon atoms, an alkyl having 1 to 3 carbon atoms, and the like. Examples include methyl, ethyl, n-propyl, isopropyl, n-butyl, isobutyl, sec-butyl, tert-butyl, n-pentyl, isopentyl, neopentyl, hexyl, isohexyl and the like.

The term "C1-C3 alkyl" means a linear or branched alkyl having 1 to 3 carbon atoms. Examples include methyl, ethyl, n-propyl, isopropyl and the like.

For "C1-C6 alkyl" in "substituted or unsubstituted C1-C6 alkyl" of R¹³, "C1-C3 alkyl" is preferable. Example include methyl, ethyl, n-propyl, isopropyl and the like, and methyl is particularly preferred.

Examples of the substituent in "substituted or unsubstituted C1-C6 alkyl" of R¹³ include halogen, an aromatic carbocyclyl optionally substituted with one or more substituents selected from the group consisting of Substituent Group α (Substituent Group α: halogen, cyano, C1-C6 alkyl, halo C1-C6 alkyl, C1-C6 alkyloxy and halo C1-C6 alkyloxy), an aromatic heterocyclyl optionally substituted with one or more substituents selected from the group consisting of Substituent Group α, a non-aromatic carbocyclyl optionally substituted with one or more substituents selected from the group consisting of Substituent Group α, a non-aromatic heterocyclyl optionally substituted with one or more substituents selected from the group consisting of Substituent Group α. An aromatic carbocyclyl optionally substituted with one or more substituents selected from the group consisting of the Substituent Group α is preferable. An aromatic carbocyclyl is preferable, and phenyl is particularly preferable.

The term "halo C1-C6 alkyl" means "C1-C6 alkyl" described above substituted with one or more halogens, and examples include monotrifluoromethyl, ditrifluoromethyl, trifluoromethyl, trifluoroethyl, trifluoropropyl, trifluorobutyl, trifluoropentyl, trifluorohexyl and the like.

The term "halo C1-C3 alkyl" means "C1-C3 alkyl" described above substituted with one or more halogens, and examples include trifluoromethyl and trifluoroethyl.

The term "C1-C6 alkylamino" means an amino group substituted with one or two "alkyl" described above on the nitrogen of the amino group, and the two alkyls may the same or different. Examples include methylamino, ethylamino, dimethylamino, diethylamino and the like.

The term "carbocyclyl" means "aromatic carbocyclyl" or "non-aromatic carbocyclyl".

The term "carbocycle" means a ring derived from "carbocyclyl" described above.

The term "heterocyclyl" means "aromatic heterocyclyl" or "non-aromatic heterocyclyl".

The "heterocycle" means a ring derived from "heterocyclyl" described above.

The term "aromatic carbocyclyl" means a monocyclic or polycyclic aromatic hydrocarbon group. Examples include phenyl, naphthyl, anthryl, phenanthryl and the like.

A preferred embodiment of "aromatic carbocyclyl" is phenyl or naphtyl.

The term "aromatic carbocycle" means a ring derived from "aromatic carbocyclyl" described above.

The term "non-aromatic carbocyclyl" means a monocyclic or polycyclic saturated hydrocarbon group or unsaturated non-aromatic hydrocarbon group. The "non-aromatic carbocyclyl" which is polycyclic includes a fused ring group wherein a non-aromatic carbocyclyl, which is monocyclic or polycyclic, is fused with a ring of the above "aromatic carbocyclyl".

In addition, examples of the "non-aromatic carbocyclyl" also include a group having a bridge or a group to form a spiro ring as follows:

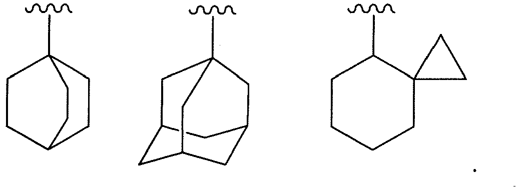

The non-aromatic carbocyclyl which is monocyclic is preferably C3 to C16, more preferably C3 to C12 and further preferably C4 to C8 carbocyclyl. Examples include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, cyclohexadienyl and the like.

Examples of non-aromatic carbocyclyl, which is polycyclic, is preferably C8 to C20 and more preferably C8 to C16 carbocyclyl. Examples include indanyl, indenyl, acenaphthyl, tetrahydronaphthyl, fluorenyl and the like.

The term "non-aromatic carbocycle" means a ring derived from "non-aromatic carbocyclyl" described above.

The term "aromatic heterocyclyl" means an aromatic cyclyl, which is monocyclic or polycyclic, containing one or more, same or different heteroatom(s) selected independently from O, S and N.

The aromatic heterocyclyl, which is polycyclic, includes a fused ring group wherein an aromatic heterocyclyl, which is monocyclic or polycyclic, is fused with a ring of the above "aromatic carbocyclyl". The free valency from the "heterocyclyl" may be on either ring.

The aromatic heterocyclyl, which is monocyclic, is preferably 5- to 8-membered, more preferably 5- to 6-membered aromatic heterocyclyl. Examples of the 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of the 6-membered aromatic heterocyclyl include pyridyl, pyridazinyl, pyrimidinyl, pyrazinyl, and triazinyl.

The aromatic heterocyclyl, which is bicyclic, is preferably 8- to 10-membered, more preferably 9- or 10-membered aromatic heterocyclyl. Examples thereof include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl.

The aromatic heterocyclyl, which is polycyclic having three or more rings, is preferably 13- to 15-membered aromatic heterocyclyl. Examples thereof include carbazolyl, acridinyl, xanthenyl, phenothiazinyl, phenoxathiinyl, phenoxazinyl, and dibenzofuryl.

The term "aromatic heterocycle" means a ring from "aromatic heterocyclyl" described above.

The term "non-aromatic heterocyclyl" means non-aromatic cyclyl, which is monocyclic or polycyclic having two or more rings, containing one or more and same or different heteroatoms selected independently from O, S and N in the ring. The "non-aromatic heterocyclyl", which is polycyclic, include an above-mentioned non-aromatic heterocyclyl, which is monocyclic or polycyclic, fused with a ring of the above "aromatic carbocyclyl", "non-aromatic carbocyclyl" and/or "aromatic heterocyclyl" and also include an above-mentioned non-aromatic heterocyclyl, which is monocyclic or polycyclic, fused with a ring of the above "aromatic heterocyclyl", and the free valency may be on either ring.

In addition, Examples of the "non-aromatic heterocyclyl" includes a group having a bridge or a group to form a spiro ring as follows:

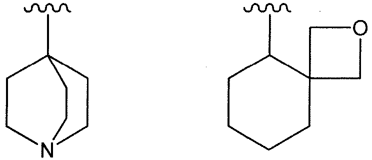

The non-aromatic heterocyclyl, which is monocyclic, is preferably 3- to 8-membered and more preferably 5- to 6-membered. Examples of non-aromatic heterocyclyl, which is 3-membered, include thiiranyl, oxiranyl and aziridinyl. Examples of non-aromatic heterocyclyl, which is 4-membered, include oxetanyl and azetidinyl. Examples of non-aromatic heterocyclyl, which is 5-membered, include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl, thiolanyl and the like. Examples of non-aromatic heterocyclyl, which is 6-membered, include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydrooxazinyl, tetrahydropyridazinyl, hexahydropyrimidinyl, dioxazinyl, thiynyl, thiazinyl and the like. Examples of non-aromatic heterocyclyl, which is 7-membered, include hexahydroazepinyl, tetrahydrodiazepinyl and oxepanyl.

The non-aromatic heterocyclyl, which is polycyclic, is preferably 8- to 20-membered and more preferably 8- to 10-membered. Examples include indolinyl, isoindolinyl, chromanyl, isochromanyl and the like.

The term "non-aromatic heterocycle" means a ring derived from "non-aromatic heterocyclyl" described above.

The term "hydroxyl protecting group" means a group replacing hydrogen of a hydroxyl group, and a group which is deprotected by a general method, such as described in Protective Groups in Organic Synthesis, Theodora W Green (John Wiley & Sons), to generate hydroxyl group. Examples include aromatic carbocyclylalkyl optionally substituted by a group selected from Substituent Group B (e.g., benzyl, p-methoxyphenylbenzyl), alkylcarbonyl optionally substituted by a group selected from Substituent Group A (e.g., acetyl, pivaloyl, chloroacetyl), formyl, aromatic carbocyclylcarbonyl optionally substituted by a group selected from Substituent Group B (e.g., benzoyl), alkyloxycarbonyl optionally substituted by a group selected from Substituent Group A (e.g., methoxycarbonyl, isobutyloxycarbonyl, benzyloxycarbonyl, vinyloxycarbonyl), aromatic carbocyclyloxycarbonyl optionally substituted by a group selected from Substituent Group B (e.g., phenyloxycarbonyl), alkylsulfonyl optionally substituted by a group selected from Substituent Group A (e.g., mesyl), aromatic carbocyclylsulfonyl optionally substituted by a group selected from Substituent Group B (e.g., tosyl), trialkylsilyl (e.g., trimethylsilyl, triethylsilyl, t-butyldimethylsilyl), alkyloxyalkyl optionally substituted by a group selected from Substituent Group A (e.g., methoxymethyl, benzyloxymethyl, methoxyethoxymethyl), 2-(trimethylsilyl)ethoxymethyl, propenyl, phenacyl, tetrahydropyranyl, alkyl, and the like.

Substituent Group A: halogen, amino, alkylamino, alkylsulfonyl, aromatic carbocyclic sulfonyl, alkylsulfinyl, aromatic carbocyclic sulfinyl, nitro, alkyloxy, alkyloxycarbonyl, alkylcarbamoyl and aromatic carbocyclyl.

Substituent Group B: halogen, amino, alkylamino, alkylsulfonyl, aromatic carbocyclic sulfonyl, alkylsulfinyl, aromatic carbocyclic sulfinyl, nitro, alkyl, haloalkyl, alkyloxy, alkyloxycarbonyl, alkylcarbamoyl and aromatic carbocyclyl.

The term "optionally substituted by a group selected from Substituent Group A" means that any position may be substituted by one, two or more same or different groups selected from Substituent Group A.

The same applies to the terms "optionally substituted by a group selected from Substituent Group B".

The term "removable functional group" means a leaving group or a functional group which can be converted into a leaving group. Examples include optionally protected carboxy, optionally protected amino, optionally protected hydroxy, chlorine, bromine, iodine, or silyl-type functional group. Preferred is optionally protected carboxy, and more preferred is protected carboxy.

The term "optionally protected carboxy" means carboxy or a group which can be deprotected according to a general method, such as described in Protective Groups in Organic Synthesis, Theodora W Green (John Wiley & Sons), to generate carboxy group. Preferred is alkyloxycarbonyl optionally substituted by a group selected from Substituent Group A (e.g., methyloxycarbonyl, ethyloxycarbonyl).

The "optionally protected carboxy" may be deprotected to carboxy, which is then removed, or may be converted into "active ester", which is then removed.

Examples of the "active ester" include those already reported, particularly, as an ester having high desorption ability, a group of the formula: -C(=O)-O-R²⁰ wherein R²⁰ is a group of the formula: 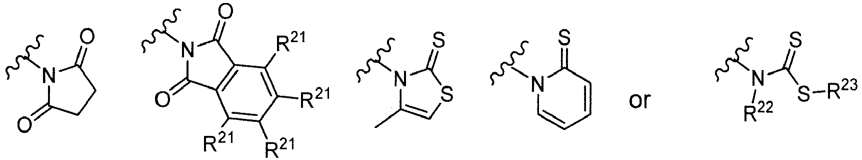 wherein R²¹ is each independently hydrogen or halogen; R²² is alkyl optionally substituted by a group selected from Substituent Group A or aromatic carbocyclyl optionally substituted by a group selected from Substituent Group B; R²³ is alkyl optionally substituted by a group selected from Substituent Group A or aromatic carbocyclyl optionally substituted by a group selected from Substituent Group B.

More preferred is a group of the following formula: 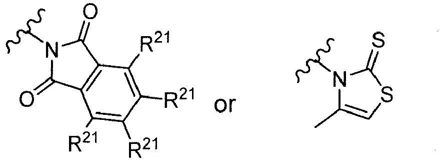 wherein each symbol is as defined above.

"Silyl-type functional group" may be any group as long as it can be removed by fluoride ion reagent. Examples include a group of the formula: -Si(R^{A4})₃ wherein R^{A4} is each independently alkyl optionally substituted by a group selected from Substituent Group A or aromatic carbocyclyl optionally substituted by a group selected from Substituent Group B.

The term "acrydinium salt" is a salt of a compound of the formula (X): 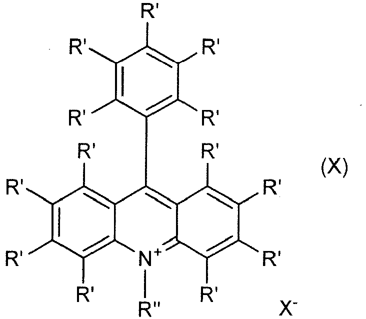 wherein
R' is independently hydrogen, halogen, C1-C6 alkyl, halo C1-C6 alkyl, C1-C6 alkyloxy, halo C1-C6 alkyloxy or C1-C6 alkylamino;
R" is C1-C6 alkyl, C1-C6 alkyl substituted with an aromatic carbocyclyl, C1-C6 alkyl substituted with an aromatic heterocyclyl; and
X⁻ is an anion.
The salt may be any salt so long as it does not interfere with the reaction.

The term "leaving group" includes halogen, p-toluenesulfonyl, trifluoromethanesulfonyl and methanesulfonyl. Examples include halogen.

The term "metal reagent" includes organic magnesium compounds, organic zinc compounds, organic copper compounds, and organic lithium compounds. Preferred is an organic magnesium compound, and more preferred is a Grignard reagent and an organic magnesium iodide. Particularly preferred is a Grignard reagent.

The term "monocyclic aryl" in "monocyclic aryl metal reagent" means to include a group of the formula: 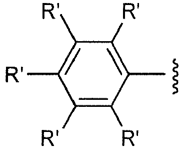 wherein R' is independently hydrogen, halogen, C1-C6 alkyl, halo C1-C6 alkyl, C1-C6 alkyloxy, halo C1-C6 alkyloxy or C1-C6 alkylamino.

In the definition of the compound of formula (IV): 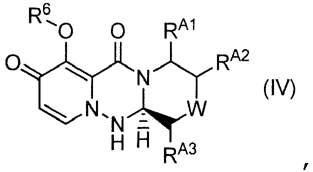 the wording "R^{A1} is hydrogen or RA; R^{A2} is hydrogen or RA; R^{A3} is hydrogen or RA; W is O, CH₂ or CHRA; RA is a removable functional group, the carbon atom to which the RA is bonded is optically active, provided that any one of R^{A1}, R^{A2} and R^{A3} is RA and the other two are hydrogen and W is O or CH₂, or R^{A1}, R^{A2} and R^{A3} are hydrogen and W is CHRA" means to include compounds of the following formulas:

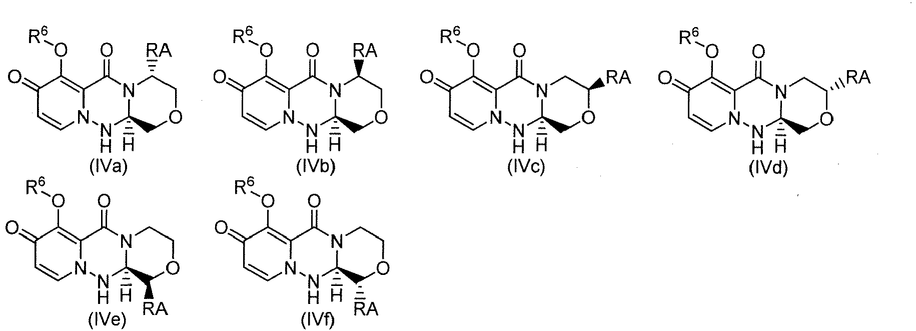

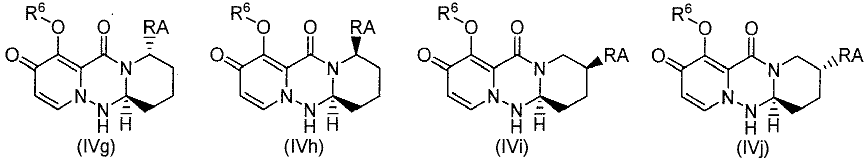

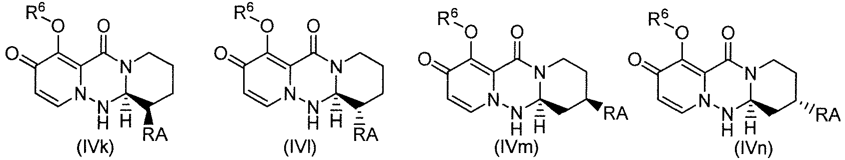

Preferred is that R^{A1} is RA, and more preferred are a compound of the formula (IVa), a compound of the formula (IVb), a compound of the formula (IVg), and a compound of the formula (IVh), and most preferred is a compound of the formula (IVa).

Preferred embodiments for each of R¹, R², R³, R⁴, R⁵, and X⁻ in the acridinium salt of the formula (I): 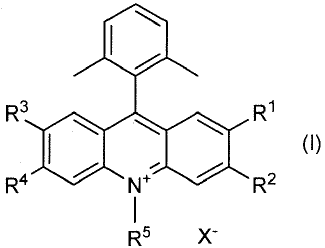 are described below. Examples of the acridinium salt of the formula (I) include all combinations of the embodiments described below.

According to one embodiment, examples of R¹ include C1-C6 alkyl and C1-C6 alkyloxy.

According to a preferred embodiment, examples of R¹ include C1-C6 alkyl.

According to a more preferred embodiment, examples of R¹ include C1-C3 alkyl.

According to another preferred embodiment, examples of R¹ include C1-C6 alkyloxy.

According to a more preferred embodiment, examples of R¹ include C1-C3 alkyloxy.

According to a more preferred embodiment, examples of R¹ include C1-C3 alkyl and C1-C3 alkyloxy.

According to a particularly preferred embodiment, examples of R¹ include methyloxy and ethyloxy.

According to one embodiment, examples of R² include hydrogen and C1-C6 alkyloxy.

According to a preferred embodiment, examples of R² include hydrogen.

According to another preferred embodiment, examples of R² include C1-C6 alkyloxy.

According to a more preferred embodiment, examples of R² include C1-C3 alkyloxy.

According to a particularly preferred embodiment, examples of R² include hydrogen, methyloxy and isopropyloxy.

According to one embodiment, examples of R³ include hydrogen, halogen, C1-C6 alkyl and C1-C6 alkyloxy.

According to a preferred embodiment, examples of R³ include hydrogen, halogen, C1-C3 alkyl and C1-C3 alkyloxy.

According to a more preferred embodiment, examples of R³ include hydrogen, halogen, isopropyl, t-butyl and methyloxy.

According to a particularly preferred embodiment, examples of R³ include hydrogen.

According to one embodiment, examples of R⁴ include hydrogen, C1-C6 alkyloxy, halo C1-C6 alkyloxy and C1-C6 alkylamino.

According to a preferred embodiment, examples of R⁴ include hydrogen, halogen, C1-C3 alkyl and C1-C3 alkyloxy.

According to a more preferred embodiment, examples of R⁴ include halogen, methyloxy, isopropyloxy, haloethyloxy and dimethylamino.

According to a particularly preferred embodiment, examples of R⁴ include methyloxy and isopropyloxy.

According to one embodiment, examples of R⁵ include C1-C3 alkyl.

According to a preferred embodiment, examples of R⁵ include methyl, ethyl and propyl.

According to a more preferred embodiment, examples of R⁵ include methyl.

According to one embodiment, examples of X⁻ include anions.

According to a preferred embodiment, examples of X⁻ include BF₄⁻, ClO₄⁻ and Cl⁻.

According to a more preferred embodiment, examples of X⁻ include BF₄⁻ and ClO₄⁻.

According to a particularly preferred embodiment, examples of X⁻ include BF₄⁻.

Preferred embodiments for each of R⁷ to R¹², R¹³, Y and Z in the compound of the formula (II): 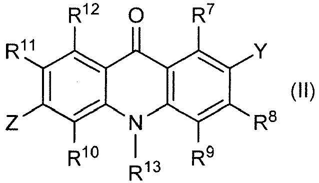 are described below. Examples of the compound of the formula (II) include all combinations of the embodiments described below.

According to one embodiment, examples of R⁷ to R¹² include, independently, hydrogen, C1-C6 alkyloxy, halo C1-C6 alkyloxy and C1-C6 alkylamino.

According to a preferred embodiment, examples of R⁷ to R¹² include, independently, hydrogen, C1-C3 alkyloxy, halo C1-C3 alkyloxy and C1-C3 alkylamino.

According to a more preferred embodiment, examples of R⁷ to R¹² include, independently, hydrogen, methyloxy, ethyloxy, isopropyloxy, halomethyloxy, haloethyloxy, and methylamino.

According to another embodiment, examples of R⁷, R⁹, R¹⁰, R¹¹ and R¹² include hydrogens, and examples of R⁸ include hydrogen, methyloxy and isopropyloxy.

According to one embodiment, examples of R¹³ include substituted or unsubstituted C1-C6 alkyl and substituted or unsubstituted aromatic carbocyclyl.

According to a preferred embodiment, examples of R¹³ include C1-C6 alkyl.

According to a more preferred embodiment, examples of R¹³ include C1-C3 alkyl.

According to a particularly preferred embodiment, examples of R¹³ include methyl.

According to one embodiment, examples of Y include C1-C6 alkyloxy.

According to a preferred embodiment, examples of Y include C1-C3 alkyloxy.

According to a more preferred embodiment, examples of Y include methyloxy, ethyloxy and isopropyloxy.

According to one embodiment, examples of Z include leaving groups.

According to one embodiment, examples of Z include halogen, p-toluenesulfonyl, trifluoromethanesulfonyl and methanesulfonyl.

Preferred embodiments for each of R⁷ to R¹², R¹³, Y, Z and R¹⁴ to R¹⁸ in the acridinium salt of the formula (III): 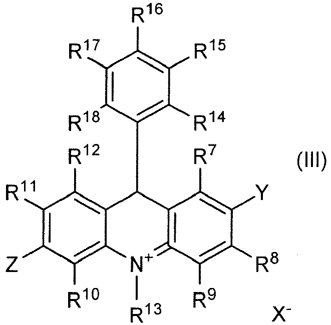 are described below. Examples of the acridinium salt of the formula (III) include all combinations of the embodiments described below. R⁷ to R¹², R¹³ and Y are as defined in the formula (II).

According to one embodiment, examples of R¹⁴ to R¹⁸ include, independently, hydrogen, halogen, C1-C6 alkyloxy, halo C1-C6 alkyloxy and C1-C6 alkylamino.

According to a preferred embodiment, examples of R¹⁴ to R¹⁸ include, independently, hydrogen and C1-C3 alkyl.

According to a more preferred embodiment, examples of R¹⁴ and R¹⁸ include, independently, C1-C3 alkyl, examples of R¹⁵ and R¹⁷ include hydrogen, and examples of R¹⁶ include hydrogen and C1-C3 alkyl.

According to a particularly embodiment, examples of R¹⁴ to R¹⁸ include methyl, and examples of R¹⁵ to R¹⁷ include hydrogen.

General procedures of the process of the present invention are represented by the following schemes.

As used herein, "solid wedge line" and "dashed wedge line" indicate absolute configuration.

In a reaction of a compound with another compound, as described herein, these compounds may be a salt or a solvate thereof. Moreover, the reactions as described below may be conducted "in continuous steps" without isolation. Conducting "in continuous steps" comprises performing the next step without isolation of a compound obtained by the reaction in the preceding step. For example, two steps may be carried out in one-pot.

A compound of the formula (VIIIa) or (IXa) can be used as a medicament in a form of a salt. Examples of such pharmaceutically acceptable salt of the compound of the formula (VIIIa) or (IXa) include salts with alkaline metals (e.g., lithium, sodium, potassium), alkaline earth metals (e.g., calcium, barium), magnesium, transition metals (e.g., zinc, iron), ammonia, organic bases (e.g., trimethylamine, triethylamine, dicyclohexylamine, ethanolamine, diethanolamine, triethanolamine, meglumine, ethylenediamine, pyridine, picoline, quinoline) or amino acids, or salts with inorganic acids (e.g., hydrochloric acid, sulfuric acid, nitric acid, carbonic acid, hydrobromic acid, phosphoric acid, hydroiodic acid), or organic acids (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid), particularly, salts with hydrochloric acid, sulfuric acid, phosphoric acid, tartaric acid, methanesulfonic acid and the like. These salts can be formed in accordance with the conventional methods.

The meaning of each term is as follows.
ABCN: 1,1'-azobis (cyclohexanecarbonitrile)
acac: Acetylacetonet
AIBN: Azobisisobutyronitrile
Boc: tert-butoxycarbonyl
COD: 1,5-cyclooctadiene
DABCO: 1,4-diazabicyclo[2.2.2]octane
dba: Dibenzylideneacetone
DBDMH: 1,3-dibromo-5,5-dimethylhydantoin
DBU: Diazabicycloundensen
DDDS: 4,4'-dichlorodiphenyldisulfide
DMA: N,N-dimethylacetamide
DMF: N,N-dimethylformamide
EDC: 1-ethyl-3-(3-dimethylaminopropyl)carbodiimide
HATU: O-(7-azabenzotriazole-1-yl)-N,N,N',N'-tetramethyluronium hexafluorophosphate
HF: Hydrogen fluoride
n-Hex: n-hexyl
NBS: N-Bromosuccinimide
NCS: N-Chlorosuccinimide
NIS: N-iodosuccinimide
TBAF: Tetrabutylammonium fluoride
THF: tetrahydrofuran
T3P: Propylphosphonic anhydride (cyclic trimmer)
V-70: 2,2'-azobis (4-methoxy-2,4-dimethylvaleronitrile)
WSCD • HCl: 1-ethyl-3-(3-dimethylaminopropyl) carbodiimide hydrochloride

### (Process 1)

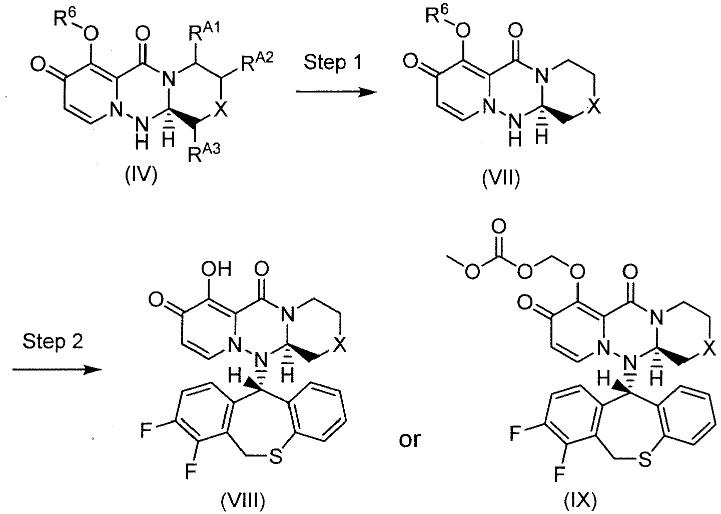

wherein each symbol is as defined above.

### Step 1

A compound of the formula (VII) is obtained by removing RA from a compound of the formula (IV).

In case that RA is an optionally protected carboxy, the compound of the formula (IV) can be deprotected to remove the protecting group under general deprotection conditions to obtain a carboxylic acid. In case that RA is carboxy, the carboxylic acid can be used as it is. The deprotection of the protecting group can be carried out according to a method, as generally described in Protective Groups in Organic Synthesis, Theodora W Green (John Wiley & Sons). The compound of the formula (VII) can be obtained by reacting the carboxylic acid with an aclidinium salt and a thiol or a disulfide, in the presence of a base under light irradiation.

The solvent is not limited as long as it allows the step to proceed efficiently. Examples include methanol, ethanol, water, dichloromethane, dichloroethane and the like. The solvent may be used alone or in combination. Preferably, a mixed solvent of ethanol and dichloromethane can be used.

Examples of the base include 2,6-lutidine, pyridine, DBU, diisopropylethylamine, triethylamine, N-methylimidazole, imidazole, DABCO and the like. Preferably, imidazole can be used.

Examples of the acridinium salt include salts of compound of the formula (X). Preferably, the acridinium salt of the formula (I) can be used. However, it is not limited as long as it allows the reaction to proceed under light irradiation.

Examples of thiol and disulfide include those as defined below, and thiol is preferable.

The disulfide is a compound having a disulfide group as a functional group, and examples include a diaromatic carbocyclic disulfide. Preferred are diphenyl disulfide or 4,4'-dichlorodiphenyl disulfide.

The thiol is a compound having a -SH group as a functional group, and examples include substituted or unsubstituted aromatic carbocyclic thiols. Preferred is 4-isopropylbenzenethiol.

The light is preferably a blue LED.

The reaction temperature may be, but not limited to, about 0°C to about 50°C, preferably room temperature.

The reaction time may be, but not limited to, 0.1 hour to 48 hours, preferably 0.1 to 12 hours.

### Step 2

A compound of the formula (VIII) or (IX) can be obtained according to the method as described in any of Patent Documents 15 to 17.

Hereinafter, the present invention will be described in more detail with reference to examples of the present invention, but the present invention is not limited thereto.

### Examples

The NMR analysis was carried out using Bruker NMR spectrometer. 1H (400 MHz) and 13C (101 MHz) NMR used the solvent peak as a reference. For 19F NMR (376 MHz), the values are as measured without using a reference.

### Example 1: production of acridinium salt I-01

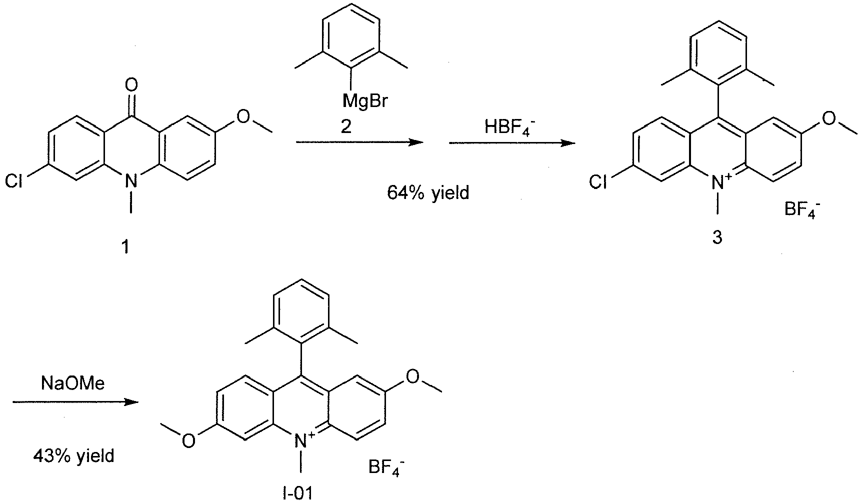

### Step 1: preparation of acridinium salt 3

A mixture of Compound 2 (200.0 mg, 0.73 mmol), THF (6 mL) and 1M THF solution of compound 2 (1.1 mL, 1.1 mmol) was stirred at 60°C for 5 hours under a nitrogen atmosphere. 1M THF solution of Compound 2 (0.1 mL, 0.1 mmol) was added, and the mixture was stirred further for 1.5 hours. After cooling the reaction solution to room temperature, 42% aqueous tetrafluoroboric acid solution (400 µL), water and dichloromethane were added to separate the solution. The aqueous layer was extracted with dichloromethane, and then the organic layer were combined and concentrated. The residue was dissolved in dichloromethane and washed with water. After concentrating the organic layer, the obtained residue was purified by silica gel column chromatography (ethyl acetate-hexane to dichloromethane-methanol), and a mixed solution of ethyl acetate and methyl tert-butyl ether was added to solidify. The solid was collected by filtration to obtain acridinium salt 3 (211.6 mg, yield 64%).
1H-NMR (CDCl₃) δ:1.79 (s, 6H), 3.78 (s, 3H), 5.00 (s, 3H), 6.84 (d, J=2.9 Hz, 1H), 7.35 (d, J=8.0 Hz, 2H), 7.50 (t, J=8.0 Hz, 1H), 7.62-7.70 (m, 2H), 8.08 (dd, J=9.9, 2.9 Hz, 1H), 8.60 (d, J=1.2 Hz, 1H)
19F-NMR (CDCl₃) δ: -153.56.
13C NMR (CDCl₃) δ: 19.97, 39.50, 56.04, 104.18, 118.37, 121.39, 124.32, 127.65, 128.44, 129.60, 129.68, 130.44, 132.16, 133.51, 136.04, 138.76, 140.11, 144.87, 158.56, 159.10.

### Step 2: Preparation of acridinium salt I-01

A mixture of aclidinium salt 3 (19.59 mg, 0.044 mmol), methanol (644 µL), and 28% sodium methoxide methanol solution (9 µL) was stirred at room temperature for 36 minutes. To the reaction solution was added 42% aqueous solution of tetrafluoroboric acid (10 µL), dichloromethane, and aqueous solution of sodium tetrafluoroborate to separate layers. The aqueous layer was extracted with dichloromethane. The combined organic layer was washed with aqueous sodium tetrafluoroborate solution, and dried over sodium sulfate. The solvent was replaced with ethyl acetate to precipitate a solid. The solvent was replaced with methyl tert-butyl ether, and the solid was collected by filtration and washed with methyl tert-butyl ether. The obtained solid was dried under reduced pressure to obtain acridinium salt I-01 (8.41 mg, yield 43%).
1H-NMR (CDCl₃) δ:1.80 (s, 6H), 3.76 (s, 3H), 4.30 (s, 3H), 4.94 (s, 3H), 6.83 (d, J=2.8 Hz, 1H), 7.29 (dd, J=9.4, 2.1 Hz, 1H), 7.33 (d, J=7.6 Hz, 2H), 7.49 (t, J=7.6 Hz, 1H), 7.54 (d, J=9.4 Hz, 1H), 7.87 (d, J=2.1 Hz, 1H), 7.94 (dd, J=9.6, 2.8 Hz, 1H), 8.55 (d, J=9.8 Hz, 1H).
19F-NMR (CDCl₃) δ:-152.39.
13C NMR (CDCl₃) δ:19.97, 39.12, 55.90, 57.79, 97.25, 105.22, 120.21, 122.25, 123.68, 125.52, 128.34, 129.66, 130.15, 130.46, 132.61, 135.89, 137.10, 143.78, 156.80, 158.13, 168.12.

### Example 2: Production of acridinium salt 1-02

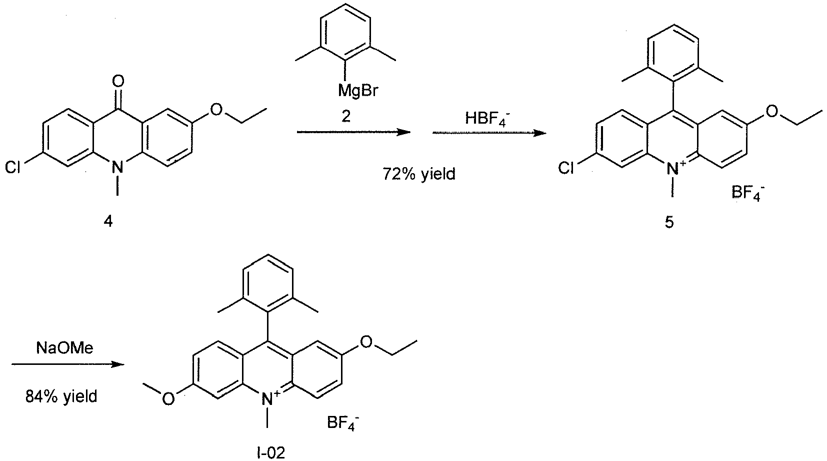

### Step 1: Preparation of acridinium salt 5

A mixture of Compound 4 (1002.1 mg, 3.5 mmol), pyridine (10 mL), and 1M THF solution of Compound 2 (13 mL, 13 mmol) was stirred at 55°C for 6 hours under a nitrogen atmosphere. 1M THF solution of Compound 2 (5 mL, 5 mmol) was added and heated at 58°C for 9 hours. After cooling the reaction solution to room temperature, formic acid (1120 µL, 30 mmol) and water (20 mL) were added, and the mixture was concentrated to 16.19 g. To the concentrated solution were added dichloromethane (15 mL), water (5 mL) and 42% aqueous tetrafluoroboric acid solution (2 mL) to separate the layers. The aqueous layer was extracted with dichloromethane (2 mL). The combined organic layer was washed with a mixed solution of water (10 mL) and 42% tetrafluoroboric acid aqueous solution (1 mL), and then with a mixed solution of water (5 mL) and sodium tetrafluoroborate (191.2 mg, 1.7 mmol). The obtained organic layer was concentrated. The solvent was replaced with ethyl acetate, and the mixture was concentrated to 8.00 g. The precipitated solid was collected by filtration and washed with ethyl acetate (5 mL). The obtained solid was dried under reduced pressure to obtain acridinim salt 5 (1170 mg, yield 72%).
1H-NMR (CDCl₃) δ:1.41 (t, J=7.0 Hz, 3H), 1.79 (s, 6H), 2.24 (s, 1H), 3.95 (q, J=7.0 Hz, 2H), 4.99 (s, 3H), 6.82 (d, J=2.9 Hz, 1H), 7.34 (d, J=7.6 Hz, 2H), 7.50 (t, J=7.6 Hz, 1H), 7.61-7.69 (m, 2H), 8.07 (dd, J=9.9, 2.9 Hz, 1H), 8.60 (d, J=1.1 Hz, 1H), 8.76 (d, J=9.9 Hz, 1H).
19F-NMR (CDCl₃) δ:-153.49.
13C NMR (CDCl₃) δ:14.21, 19.98, 39.46, 64.69, 104.74, 118.39, 121.32, 124.28, 127.70, 128.44, 129.59, 129.64, 130.42, 132.23, 133.81, 136.05, 138.65, 140.06, 144.79, 158.42, 158.45.

### Step 2: Preparation of acridinium salt I-02

A mixture of aclidinium salt 5 (6.04 g, 13.0 mmol) and methanol (240 mL) was cooled to 5°C. 28% sodium methoxide methanol solution (3.00 g, 15.5 mmol) was diluted with methanol (6 mL) and added to the mixture. After stirring at 3°C for 1 hour, the temperature was raised to 13°C, and the mixture was stirred for 1 hour. To the mixture was added 42% aqueous tetrafluoroboric acid solution (3.27 g, 15.6 mmol), and the mixture was concentrated to 62.4 g. To the mixture was added water (60 mL), and the mixture was concentrated to 73.06 g. Dichloromethane (60 mL) was added to separate the layers. The aqueous layer was extracted with dichloromethane (30 mL). The combined rganic layer was washed with a mixed solution of water (16.77 g) and sodium tetrafluoroborate (1.72 g, 15.7 mmol). The obtained organic layer was concentrated, and the solvent was replaced with ethyl acetate. After concentrating to 16.89 g, a small amount of seed crystals were added to precipitate a solid. After adding 40 mL of ethyl acetate, the mixture was concentrated to 27.52 g. The solid was collected by filtration, and washed with ethyl acetate (24 mL). The obtained solid was dried under reduced pressure to obtain acridinium salt I-02 (5.02 g, yield 84%).
1H-NMR (CDCl₃) δ:1.41 (t, J=7.0 Hz, 3H), 1.79 (s, 6H), 2.04 (s, 1H), 3.94 (q, J=7.0 Hz, 2H), 4.30 (s, 3H), 4.94 (s, 3H), 6.82 (d, J=2.9 Hz, 1H), 7.28 (dd, J=9.5, 2.3 Hz, 1H), 7.33 (d, J=7.6 Hz, 1H), 7.48 (t, J=7.6 Hz, 1H), 7.53 (d, J=9.5 Hz, 1H), 7.87 (d, J=2.3 Hz, 1H), 7.92 (dd, J=9.9, 2.9 Hz, 1H), 8.53 (d, J=9.9 Hz, 1H)
19F-NMR (CDCl₃) δ:-152.45.
13C NMR (CDCl₃) δ:14.27, 19.96, 39.08, 57.78, 64.44, 97.23, 105.82, 120.10, 122.21, 123.63, 125.56, 128.32, 129.63, 130.11, 130.73, 132.65, 135.89, 136.97, 143.71, 156.71, 157.46, 168.07.

### Example 3: Production of acridinium salt 5

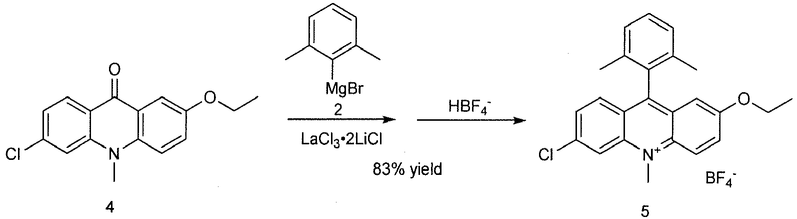

Under a nitrogen atmosphere, a mixture of Compound 4 (6.10 g, 21.20 mmol), pyridine (90 mL), 1M THF solution of Compound 2 (36 mL, 36 mmol), 0.6M THF solution of lanthanum chloride/lithium dichloride complex (3.5 mL, 2.1 mmol) was heated to 43°C for 80 minutes and then stirred at the same temperature for 230 minutes. After cooling the reaction solution to room temperature, formic acid (1.93 g, 41.9 mmol), water (49 mL) and tetrahydrofuran (50 mL) were added, and the mixture was concentrated to 63.84 g. To the concentrated mixture was added water (50 mL), and the mixture was concentrated to 59.33 g. To the concentrated mixture was added water (50 mL), and the mixture was concentrated to 93.71 g. To the concentrated mixture were added dichloromethane (70 mL), water (50 mL) and concentrated hydrochloric acid (12 mL) to separate the layers. The aqueous layer was extracted with dichloromethane (50 mL), and the combined organic layer was washed with water (50 mL). The obtained organic layer was washed twice with a mixed solution of water (50 mL) and sodium tetrafluoroborate (2.33 g, 21.2 mmol), and washed once with a mixed solution of water (25 mL) and sodium tetrafluoroborate (1.21 g, 11.0 mmol). The obtained organic layer was dried over sodium sulfate (25.01 g), and the solid was filtered, and concentrated. The solvent was replaced with ethyl acetate, and the solution was concentrated to 40.90 g. The precipitated solid was collected by filtration and washed with ethyl acetate (42 mL). The obtained solid was dried under reduced pressure to obtain acridinium salt 5 (8.20 g, yield 83%).

### Example 4 Production of acridinium salt 8

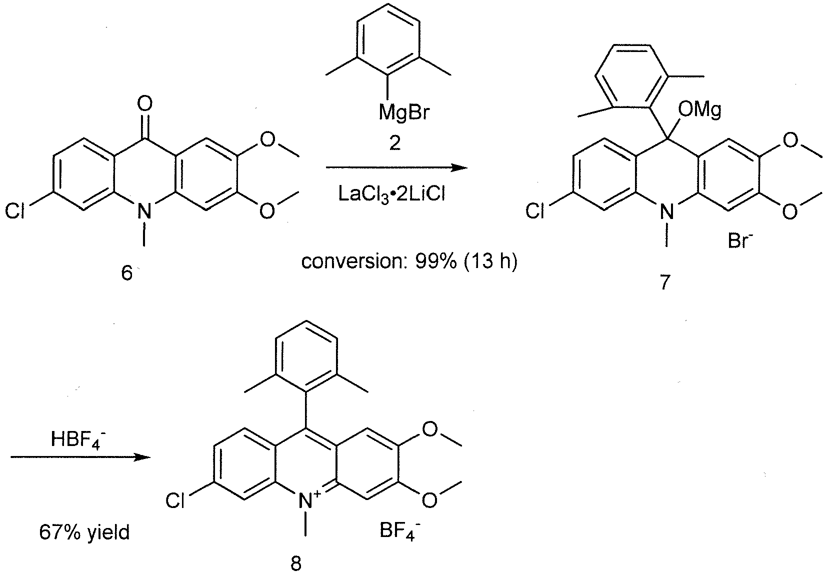

Under a nitrogen atmosphere, a mixture of Compound 6 (154.2 mg, 0.51 mmol), pyridine (3.85 mL), 1M THF solution of Compound 2 (1 mL, 1 mmol), 0.6M THF solution of lanthanum chloride/lithium dichloride complex (0.25 mL, 0.15 mmol) was stirred at 40°C for 13 hours. After cooling the mixture to room temperature, formic acid (0.2 mL) and water were added. The mixture was concentrated, and the solvent was replaced with water. To the concentrated mixture were added dichloromethane and concentrated hydrochloric acid to separate the layers. The aqueous layer was extracted with dichloromethane, and the combined organic layer was washed with an aqueous solution of tetrafluoroboric acid and with an aqueous solution of sodium tetrafluoroborate. The obtained organic layer was concentrated, ethyl acetate (2 mL) and methyl tert-butyl ether (1.5 mL) were added. The precipitated solid was collected by filtration and washed with a mixture of ethyl acetate and methyl tert-butyl ether (1:1). The obtained solid was dried under reduced pressure to obtain acridinium salt 8 (162.7 mg, yield 67%).
1H-NMR (CDCl₃) δ:1.80 (s, 6H), 3.77 (s, 3H), 4.43 (s, 3H), 4.90 (s, 3H), 6.70 (s, 1H), 7.35 (d, J=7.9 Hz, 2H), 7.50 (t, J=7.9 Hz, 1H), 7.60 (d, J=1.1 Hz, 2H), 8.01 (s, 1H), 8.43 (t, J=1.1 Hz, 1H).
19F-NMR (CDCl₃) δ:-152.26.
13C NMR (CDCl₃) δ:19.94, 39.36, 56.39, 58.89, 98.80, 103.59, 117.72, 122.58, 122.98, 128.47, 129.55, 130.38, 132.26, 135.87, 139.48, 142.33, 143.24, 151.88, 155.44, 162.44.

### Example 5: Production of acridinium salt I-03

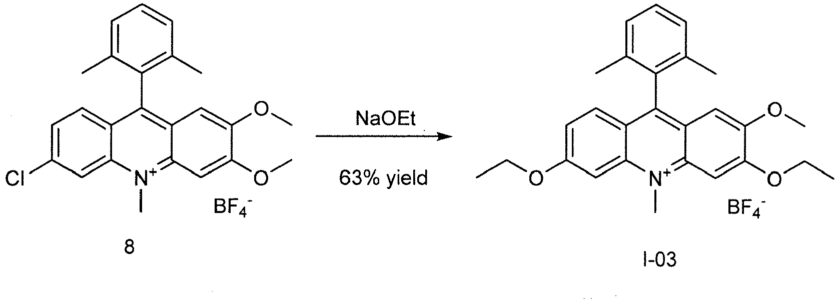

A mixed solution of acridinium salt 8 (96.9 mg, 0.20 mmol), ethanol (2 mL) and 20% sodium ethoxide solution (148 µL) was stirred under ice-cooling for 3 hours. To the mixture were added 42% tetrafluoroborate aqueous solution (90 µL), dichloromethane and activated carbon, and the mixture was filtered. The solvent of the filtrate was replaced with water. The precipitated crystals were dissolved in dichloromethane, and purified by silica gel column chromatography (dichloromethane-methanol). A mixed solution of ethyl acetate and methyl tert-butyl ether was added to solidify. The solid was collected by filtration to obtain acridinium salt I-03 (64.0 mg, yield 63%).
1H-NMR (CDCl₃) δ:1.58 (t, J=7.1 Hz,3H), 1.65(t, J=7.1 Hz,3H), 1.80 (s, 6H), 3.74 (s, 3H), 4.46 (q, J=7.1 Hz, 2H), 4.61 (q, J=7.1 Hz, 2H), 4.80 (s, 3H), 6.68 (s, 1H), 7.22 (dd, J=9.4, 2.1 Hz, 1H), 7.32 (d, J=7.6 Hz, 2H), 7.44-7.51 (m, 2H), 7.64 (d, J=2.1 Hz, 1H), 7.78 (s, 1H)
19F-NMR (CDCl₃) δ:-152.49.
13C NMR (CDCl₃) δ:14.31, 14.43, 19.90, 39.00, 56.20, 65.94, 67.18, 97.68, 98.59, 104.26, 119.91, 120.24, 121.55, 128.29, 129.64, 129.98, 132.86, 135.85, 140.66, 142.68, 150.68, 154.58, 159.79, 166.05.

The following acridinium salts were also obtained by the process described above.

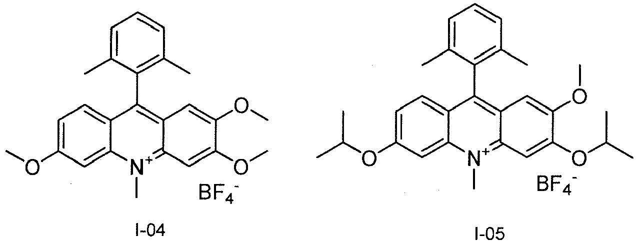

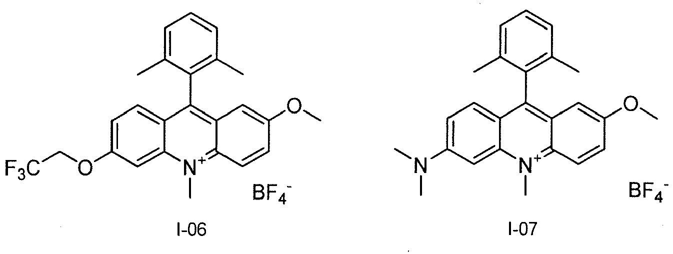

### Aclidinium salt I-04

1H-NMR (CDCl₃) δ:1.80 (s, 6H), 3.75 (s, 3H), 4.21 (s, 3H), 4.35 (s, 3H), 4.84 (s, 3H), 6.70 (s, 1H), 7.33 (d, J=7.6 Hz, 2H), 7.47 (t, J=7.6 Hz, 1H), 7.49 (s, 1H), 7.52 (s, 1H), 7.67 (d, J=2.2 Hz, 1H), 7.82 (s, 1H).

19F-NMR (CDCl₃) δ:-152.32.

### Aclidinium salt I-05

1H-NMR (acetone-d6)5:1.49 (d, J=6.0 Hz, 6H), 1.54 (d, J=5.8 Hz, 6H), 1.85 (s, 6H), 3.76 (s, 3H), 4.87 (s, 3H), 5.24 (sept, J=6.0 Hz, 1H), 5.24 (sept, J=5.8 Hz, 1H), 6.87 (s, 1H), 7.41 (d, J=7.7 Hz, 1H), 7.45 (dd, J=9.4, 2.2 Hz, 1H), 7.54 (t, J=7.7 Hz, 1H), 7.61 (d, J=9.4 Hz, 1H), 7.92 (d, J=2.2 Hz, 1H), 7.97 (s, 1H).

19F-NMR (acetone-d6)δ:-151.96.

### Aclidinium salt I-06

1H-NMR (CDCl₃) δ:1.79 (s, 6H), 3.77 (s, 3H), 4.98 (s, 3H), 5.02 (q, J=8.0 Hz, 1H), 6.85 (d, J=3.0 Hz, 1H), 7.35 (d, J=7.6 Hz, 2H), 7.40 (dd, J=9.5, 2.1 Hz, 1H), 7.50 (t, J=7.6 Hz, 1H), 7.60 (d, J=9.5 Hz, 1H), 7.95 (dd, J=9.7, 3.0 Hz, 1H), 8.07 (d, J=2.1 Hz, 1H), 8.50 (d, J=9.7 Hz, 1H).

19F-NMR (CDCl₃) δ:-73.42, -151.65.

### Aclidinium salt I-07

1H-NMR (CDCl₃) δ:1.82 (s, 6H), 3.43 (s, 6H), 3.72 (s, 3H), 4.60 (s, 3H), 5.02 (q, J=8.0 Hz, 1H), 6.73 (d, J=3.0 Hz, 1H), 7.07 (d, J=2.4 Hz, 1H), 7.19 (dd, J=9.6, 2.0 Hz, 1H), 7.29 (brd, J=7.6 Hz, 2H), 7.36 (d, J=9.6 Hz, 1H), 7.44 (t, J=7.6 Hz, 1H), 7.70 (dd, J=9.8, 3.0 Hz, 1H), 8.19 (d, J=9.8 Hz, 1H).

19F-NMR (CDCl₃) δ:-152.82.

The following compounds were obtained according to the method as described in J. AM. CHEM. SOC. 2004, 126, 15999.

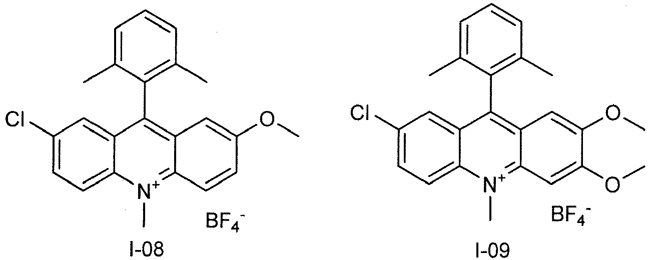

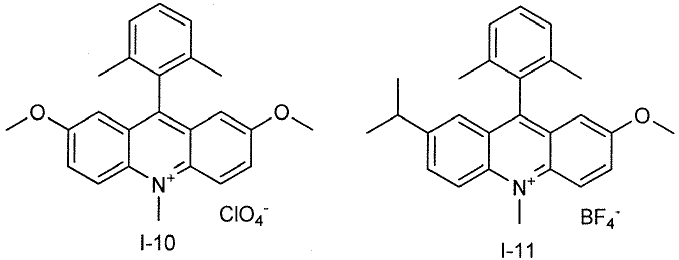

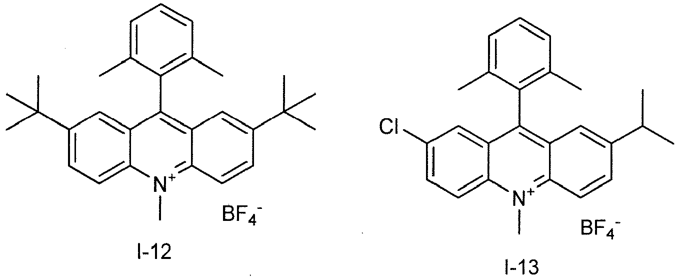

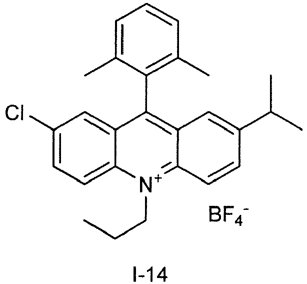

### Aclidinium salt I-08

1H-NMR (CDCl₃) δ:1.80 (s, 6H), 3.78 (s, 3H), 5.07 (s, 3H), 6.82 (d, J=2.8 Hz, 1H), 7.37 (d, J=7.4 Hz, 2H), 7.53 (t, J=7.4 Hz, 1H), 7.64 (d, J=2.2 Hz, 1H), 8.07 (dd, J=10.0, 2.8 Hz, 1H), 8.20 (dd, J=10.0, 2.2 Hz, 1H), 8.72 (dd, J=10.0, 4.8 Hz, 2 H).

19F-NMR (CDCl₃) δ:-153.32.

### Aclidinium salt I-09

1H-NMR (CDCl₃) δ:1.81 (s, 6H), 3.78 (s, 3H), 4.43 (s, 3H), 4.94 (s, 3H), 6.68 (s, 1H), 7.36 (d, J=7.8 Hz, 2H), 7.52 (t, J=7.8 Hz, 1H), 7.58 (d, J=2.4 Hz, 1H), 7.98 (s, 1H), 8.08 (dd, J=9.8, 2.4 Hz, 1H), 8.43 (d, J=9.8 Hz, 1H).

19F-NMR (CDCl₃) δ:-152.31.

### Aclidinium salt I-10

1H-NMR (CDCl₃) δ:1.81 (s, 6H), 3.77 (s, 6H), 5.06 (s, 3H), 6.79 (d, J=2.8 Hz, 2H), 7.35 (d, J=7.7 Hz, 2H), 7.50 (t, J=7.7 Hz, 1H), 7.95 (dd, J=9.9, 2.8 Hz, 2H), 8.62 (d, J=9.9 Hz, 2 H).

### Aclidinium salt I-11

Retention time of HPLC: 6.1 minutes

### Measurement conditions

Column: XSelect CSH C18 (3.5 um, i.d. 4.6 x 100 mm) (Waters) Flow rate: 1.2 mL/min; UV detection wavelength: 254 nm; Column oven temperature: 37°C; Mobile phase: [A] = 0.1% aqueous formic acid and [B] = acetonitrile.

Granant: linear gradient of 15% to 40% solvent [A] over 4 minutes, followed by 40% to 95% solvent [B] over 2 minutes, and then 95% solvent [B] was maintained for 1.5 minutes.

### Aclidinium salt I-12

1H-NMR (CDCl₃) δ:1.31 (s, 18H), 1.77 (s, 6H), 5.04 (s, 3H), 7.36 (d, J=7.6 Hz, 2H), 7.52 (t, J=7.6 Hz, 1H), 7.62 (d, J=2.2 Hz, 2H), 8.44 (dd, J=9.6, 2.2 Hz, 2H), 8.70 (d, J=9.6 Hz, 2 H).

19F-NMR (CDCl₃) δ:-153.32.

### Aclidinium salt I-13

1H-NMR (CDCl₃) δ:1.27 (d, J=6.8 Hz, 6H), 1.78 (s, 6H), 3.09 (sept, J=6.8 Hz, 1H), 5.08 (s, 1H), 7.37 (d, J=7.6 Hz, 2H), 7.49 (d, J=2.1 Hz, 1H), 7.54 (t, J=7.5 Hz, 1H), 7.69 (d, J=2.1 Hz, 1H), 8.26 (dd, J=9.6, 2.5 Hz, 1H), 8.32 (dd, J=9.5, 2.1 Hz, 1H), 8.72 (d, J=9.5 Hz, 1H), 8.78 (d, J=9.6 Hz, 1H). 19F-NMR (CDCl₃) δ:-153.25.

### Aclidinium salt I-14

1H-NMR (CDCl₃) δ:1.27 (d, J=7.0 Hz, 6H), 1.33 (t, J=7.4 Hz, 3H), 1.78 (s, 6H), 2.24-2.35 (m, 2H), 3.08 (sept, J=7.0 Hz, 1H), 5.52 (t, J=8.2 Hz, 2H), 7.37 (d, J=7.7 Hz, 2H), 7.48 (d, J=2.4 Hz, 1H), 7.54 (t, J=7.7 Hz, 1H), 7.68 (d, J=2.3 Hz, 1H), 8.28 (dd, J=9.6, 2.4 Hz, 1H), 8.33 (dd, J=9.5, 2.3 Hz, 1H), 8.63 (d, J=9.6 Hz, 1H), 8.70 (d, J=9.5 Hz, 1H).

19F-NMR (CDCl₃) δ:-153.32.

### Example 6: Elimination reaction in the presence of acridinium salt

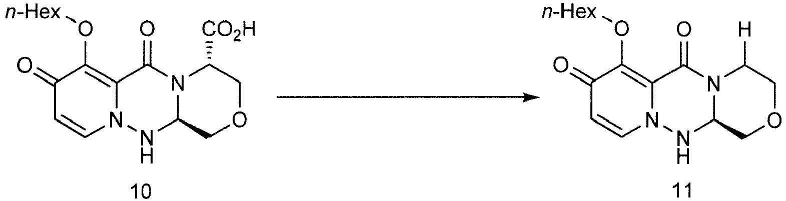

### Example 6-1

Under nitrogen atmosphere, Compound 10 (19.63 mg, 0.054 mmol), 4-isopropylbenzenethiol (0.8 mg, 5 µmol), 9-xylyl-2,7-dimethoxy-10-methylacrydinium tetrafluoroborate (0.250 mg, 0.56 µmol) and imidazole (3.752 mg, 0.055 mmol) were dissolved in a mixed solution of ethanol (50 µL) and dichloromethane (450 µL). The mixture was stirred at room temperature under light irradiation with a blue LED (power consumption 2.8 W). The conversion rate to Compound 11, as confirmed by HPLC, was 99% at 45 minutes of the reaction.

### Example 6-2

Under nitrogen atmosphere, Compound 10 (19.63 mg, 0.054 mmol), 4-isopropylbenzenethiol (0.8 mg, 5 µmol), acridinium salt I-01 (0.250 mg, 0.56 µmol), imidazole (3.752 mg, 0.055 mmol) was dissolved in a mixed solution of ethanol (50 µL) and dichloromethane (450 µL). The mixture was stirred at room temperature under light irradiation with a blue LED (power consumption 2.8 W). The conversion rate to Compound 11, as confirmed by HPLC, was 99% at 45 minutes of the reaction.

### Example 6-3

Under nitrogen atmosphere, Compound 10 (19.93 mg, 0.055 mmol), 4-isopropylbenzenethiol (0.8 mg, 5 µmol), acridinium salt 1-02 (0.264 mg, 0.57 µmol), imidazole (3.685 mg, 0.054 mmol) was dissolved in a mixed solution of ethanol (50 µL) and dichloromethane (450 µL). The mixture was stirred at room temperature under light irradiation with a blue LED (power consumption 2.8 W). The conversion rate to Compound 11, as confirmed by HPLC, was 98% at 58 minutes of the reaction. After concentrating the reaction mixture, dichloromethane (10 mL) and water (5 mL) were added to separate the layers. After concentrating the organic layer, the obtained residue was purified by silica gel column chromatography (dichloromethane-methanol) to obtain Compound 11 (16.66 mg, yield 95%).

### Example 6-4

Under nitrogen atmosphere, Compound 10 (19.60 mg, 0.054 mmol), 4-isopropylbenzenethiol (0.8 mg, 5 µmol), acridinium salt I-03 (0.263 mg, 0.55 µmol), imidazole (3.633 mg, 0.054 mmol) was dissolved in a mixed solution of ethanol (50 µL) and dichloromethane (450 µL). The mixture was stirred at room temperature under light irradiation with a blue LED (power consumption 2.8 W). The conversion rate to Compound 11, as confirmed by HPLC, was 98% at 35 minutes of the reaction.

### Example 6-5

Under nitrogen atmosphere, Compound 10 (19.84 mg, 0.054 mmol), 4-isopropylbenzenethiol (0.8 mg, 5 µmol), acridinium salt I-04 (0.300 mg, 0.56 µmol), imidazole (3.711 mg, 0.054 mmol) was dissolved in a mixed solution of ethanol (50 µL) and dichloromethane (450 µL). The mixture was stirred at room temperature under light irradiation with a blue LED (power consumption 2.8 W). The conversion rate to Compound 11, as confirmed by HPLC, was 98% at 35 minutes of the reaction.

### Reference Example

Under nitrogen atmosphere, Compound 10 (19.88 mg, 0.054 mmol), 4-isopropylbenzenethiol (0.8 mg, 5 µmol), 9-mesityl-2,7-dimethyl-10-methylacrydinium perchlorate (0.244 mg, 0.55 µmol) and imidazole (3.712 mg, 0.055 mol) were dissolved in a mixed solution of ethanol (50 µL) and dichloromethane (450 µL). The mixture was stirred at room temperature under light irradiation with a blue LED (power consumption 2.8 W). The conversion rate to Compound 11, as confirmed by HPLC, was 72% at 45 minutes of the reaction.
84% at 60 minutes of the reaction, and 97% at 90 minutes of the reaction.

### Example 7

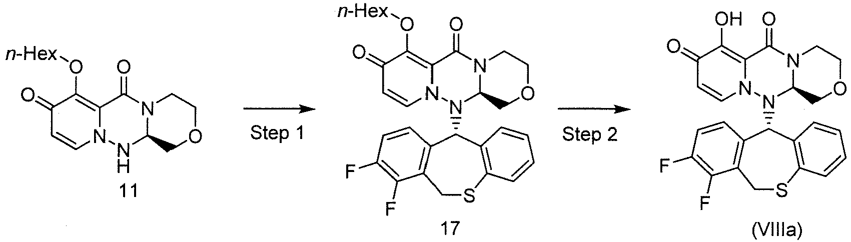

### Step 1

To compound 11 (12.0 g, 24.3 mmol) were added 7,8-difluoro-6,11-dihydrodibenzothiepine-11-ol (8.0 g, 30.3 mmol), ethyl acetate (48.7 g) and cyclohexane (14.1 g), and the mixture was stirred at 25°C. To the mixtue were added 50%(w/w) T3P ethyl acetate solution (20.91 g, 32.9 mmol) and then methanesulfonic acid (3.5 g, 36.4 mmol). The temperature was raised to 60°C, and the mixture was stirred for 24 hours. After cooling to 25°C, THF (32.0 g) and water (24.0 g) were added. To the mixture was added slowly 24% aqueous sodium hydroxide solution (30.8 g), and the mixture was allowed to stand. The layers was separated into an organic layer and an aqueous layer. The organic layer was washed twice with 7% saline (60.0 g). To the obtained solution was added a mixed solution of cyclohexane (9.3 g), ethyl acetate (32.1 g) and methanesulfonic acid (2.80 g, 29.1 mmol). The mixture was stirred at 25°C for 2 hours, and the resulting white precipitate was collected by filtration. The obtained solid was washed with ethyl acetate (43.3 g) and then dried to obtain a mesylate of compound 17 (13.65 g, yield 84.6%).
1H-NMR (DMSO-d6)δ: 0.90 (3H, t, J=6.0 Hz), 1.29-1.36 (4H, m), 1.39-1.49 (2H, m), 1.67-1.79 (2H, m), 2.38 (3H, s), 2.94 (1H, br s), 3.30 (1H, td, J=11.6, 2.4 Hz), 3.51 (1H, t, J=10.4 Hz), 3.66 (1H, dd, J=11.2, 2.8 Hz), 3.92-4.01 (2H, m), 4.07 (1H, d, J=14.3 Hz), 4.20 (1H, s), 4.42-4.52 (1H, m), 5.43 (1H, dd, J=14.4, 2.1 Hz), 5.79-5.83 (2H, m), 6.81 (1H, td, J=7.6, 1.2 Hz), 6.96 (1H, dd, J=7.8, 1.0 Hz), 7.09 (1H, J=8.0, 1.6 Hz), 7.12-7.18 (1H, m), 7.32 (1H, d, J=7.7 Hz), 7.37-7.49 (2H, m)

### Step 2

To Compound 17 (15.0 g, 22.6 mmol) was added N-methylpyrrolidone (52.4 g), and the mixture was stirred. Lithium chloride (8.6 g, 203.3 mmol) was added to the mixture, and the mixture was heated to 75°C. The mixture was stirred at 75°C for 20 hours and then cooled to 40°C. Acetonitrile (20.0 g) was added, and water (11.6 g) was further added. After cooling to 30°C and stirring for 30 minutes, water (142.5 g) was added slowly. After stirring at 30°C for 1.5 hours, the resulting white precipitate was collected by filtration. The obtained solid was washed with 2-propanol (60.1 g) and dried to obtain the compound (VIIIa) (9.91 g, yield 90.7%).

### Example 8

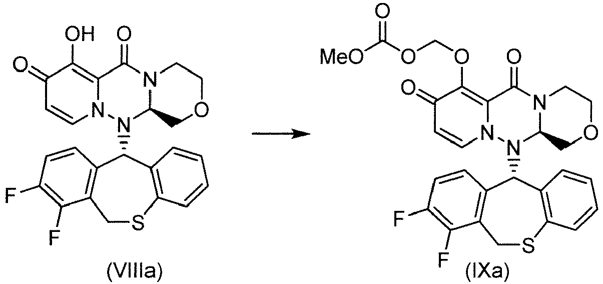

To a suspension of the compound (VIIIa) (1.00 g, 2.07 mmol) in DMA (5 ml) were added chloromethylmethylcarbonate (0.483 g, 3.10 mmol) and potassium carbonate (0.572 g, 4.14 mmol) and potassium iodide (0.343 g, 2.07 mmol), and the mixture was heated to 50°C and stirred for 6 hours. To the mixture was added further DMA (1 ml), and the mixture was stirred for 6 hours. After cooling to room temperature, DMA (6 ml) was added to the mixture, and the mixture was stirred at 50°C for 5 minutes and filtered. To the obtained filtrate were added dropwise 1 mol/L aqueous hydrochloric acid (10 ml) and water (4 ml) under ice-cooling, and the mixture was stirred for 1 hour. The precipitated solid was collected by filtration and dried under reduced pressure at 60°C for 3 hours to obtain the compound (IXa) (1.10 g, 1.93 mmol, yield 93%) .
1H-NMR (DMSO-D6) δ: 2.91-2.98 (1H, m), 3.24-3.31 (1H, m), 3.44 (1H, t, J = 10.4 Hz), 3.69 (1H, dd, J = 11.5, 2.8 Hz), 3.73 (3H, s), 4.00 (1H, dd, J = 10.8, 2.9 Hz), 4.06 (1H, d, J = 14.3 Hz), 4.40 (1H, d, J = 11.8 Hz), 4. 45 (1H, dd, J = 9.9, 2.9 Hz), 5.42 (1H, dd, J = 14.4, 1.8 Hz), 5.67 (1H, d, J = 6.5 Hz), 5.72-5.75 (3H, m), 6.83-6.87 (1H, m), 7.01 (1H, d, J = 6.9 Hz), 7.09 (1H, dd, J = 8.0, 1.1 Hz), 7.14-7.18 (1H, m), 7.23 (1H, d, J = 7.8 Hz), 7.37-7.44 (2H, m).

### Industrial Applicability

The present invention provide a catalyst useful in a process for the production of substituted polycyclic pyridone derivatives having a cap-dependent endonuclease inhibitory activity and intermediates thereof.

## Claims

1. An aclidinium salt of the formula (I): 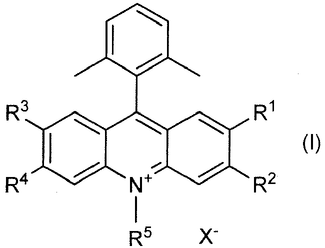 wherein
R¹ is C1-C6 alkyl or C1-C6 alkyloxy;
R² is hydrogen or C1-C6 alkyloxy;
R³ is hydrogen, halogen, C1-C6 alkyl or C1-C6 alkyloxy;
R⁴ is hydrogen, C1-C6 alkyloxy, halo C1-C6 alkyloxy or C1-C6 alkylamino;
R⁵ is C1-C3 alkyl; and
X⁻ is an anion.

2. The acridinium salt according to claim 1, wherein R¹ is C1-C3 alkyloxy.

3. The acridinium salt according to claim 1 or 2, wherein R² is hydrogen or C1-C3 alkyloxy.

4. The acridinium salt according to any one of claims 1 to 3, wherein R³ is hydrogen, halogen, C1-C3 alkyl or C1-C3 alkyloxy.

5. The acridinium salt according to any one of claims 1 to 4, wherein R⁴ is C1-C3 alkyloxy, halo C1-C3 alkyloxy or dimethylamino.

6. The acridinium salt according to any one of claims 1 to 5, wherein R⁵ is methyl.

7. The acridinium salt according to any one of claims 1 to 6, wherein R¹ is C1-C3 alkyloxy and R⁴ is C1-C3 alkyloxy.

8. The acridinium salt according to claim 1 selected from the followings: 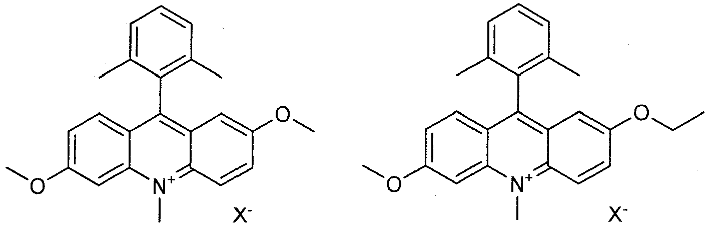 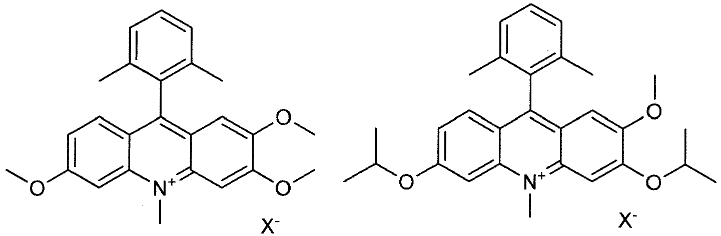 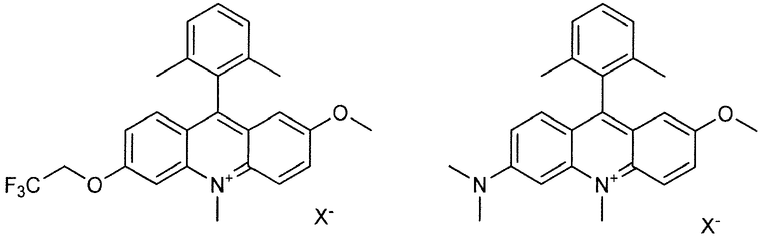 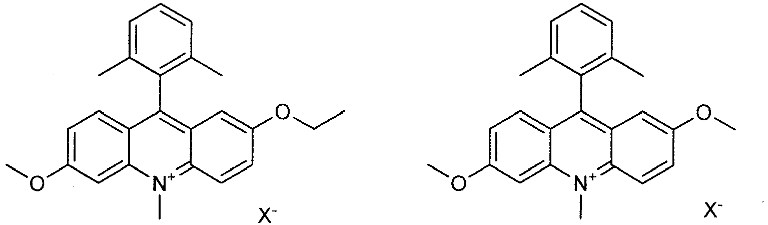 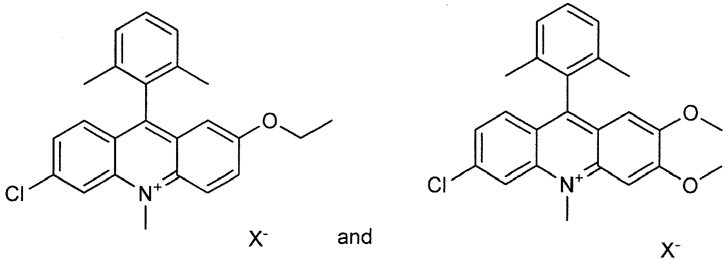 wherein X⁻ is as defined in claim 1.

9. The acridinium salt according to any one of claims 1 to 8, wherein X⁻ is BF₄⁻ or ClO₄⁻.

10. A process for the production of a compound of the formula (VII): 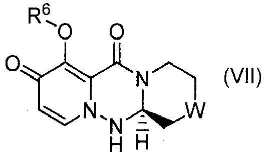
wherein W is O or CH₂ and R⁶ is hydrogen or a hydroxyl protecting group,
**characterized by** removing RA from a compound of the formula (IV): 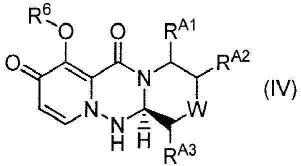 wherein
R⁶ is hydrogen or a hydroxyl protecting group;
R^{A1} is hydrogen or RA;
R^{A2} is hydrogen or RA;
R^{A3} is hydrogen or RA;
W is O, CH₂ or CHRA;
RA is a removable functional group;
the carbon atom to which the RA is bonded is optically active; and
provided that one of R^{A1}, R^{A2} and R^{A3} is RA and the other two are hydrogen and W is O or CH₂; or R^{A1}, R^{A2} and R^{A3} are hydrogen and W is CHRA,
in the presence of the acridinium salt according to any one of claims 1 to 9.

11. A process for the production of a compound of the formula (VII): 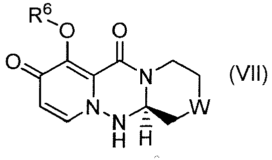
wherein W is O or CH₂ and R⁶ is hydrogen or a hydroxyl protecting group,
**characterized by** removing RA from a compound of the formula (IV): 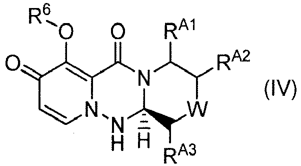 wherein
R⁶ is hydrogen or a hydroxyl protecting group;
R^{A1} is hydrogen or RA;
R^{A2} is hydrogen or RA;
R^{A3} is hydrogen or RA;
W is O, CH₂ or CHRA;
RA is a removable functional group;
the carbon atom to which the RA is bonded is optically active; and
provided that one of R^{A1}, R^{A2} and R^{A3} is RA and the other two are hydrogen and W is O or CH₂; or R^{A1}, R^{A2} and R^{A3} are hydrogen and W is CHRA,
in the presence of an acridinium salt, imidazole and 4-isopropylbenzenethiol, under light irradiation in one or more solvents selected from the group consisting of ethanol and dichloromethane.

12. The process according to claim 11, wherein RA is removed in the presence of the aclidinium salt according to any one of claims 1 to 9.

13. A process for the production of a compound of the formula (VIIIa) or formula (IXa) or a salt thereof: 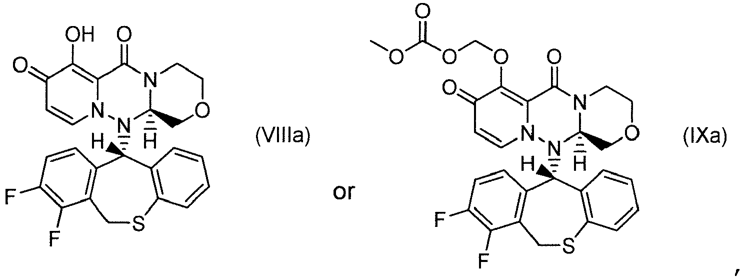 comprising the process according to any one of claims 10 to 12.

14. A process for the production of an aclidinium salt of the formula (III): 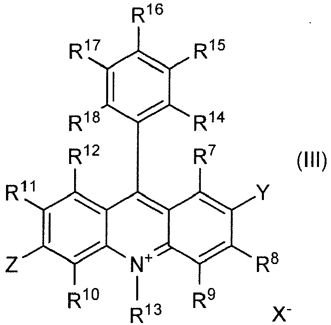 wherein
R⁷ to R¹² are independently hydrogen, C1-C6 alkyloxy, halo C1-C6 alkyloxy or C1-C6 alkylamino;
R¹³ is a substituted or unsubstituted C1-C6 alkyl or aromatic carbocyclyl;
R¹⁴ to R¹⁸ are independently hydrogen, halogen, C1-C6 alkyloxy, halo C1-C6 alkyloxy or C1-C6 alkylamino;
Y is C1-C6 alkyloxy;
Z is a leaving group; and
X⁻ is an anion,
**characterized in that** a compound of the formula (II) or a salt thereof: 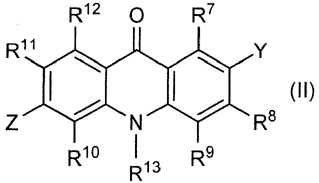 wherein
R⁷ to R¹² are independently hydrogen, C1-C6 alkyloxy, halo C1-C6 alkyloxy or C1-C6 alkylamino;
R¹³ is a substituted or unsubstituted C1-C6 alkyl or aromatic carbocyclyl;
Y is C1-C6 alkyloxy; and
Z is a leaving group,
is reacted with a monocyclic aryl metal reagent and then treated with an acid.

15. The process according to claim 14, wherein the monocyclic aryl metal reagent is a monocyclic aryl Grignard reagent.

16. A process for the production of an acridinium salt of the formula (IV): 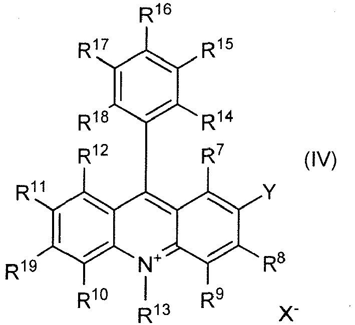 wherein
R⁷ to R¹² are independently hydrogen, C1-C6 alkyloxy, halo C1-C6 alkyloxy or C1-C6 alkylamino;
R¹³ is a substituted or unsubstituted C1-C6 alkyl or aromatic carbocyclyl;
R¹⁴ to R¹⁸ are independently hydrogen, halogen, C1-C6 alkyloxy, halo C1-C6 alkyloxy or C1-C6 alkylamino;
R¹⁹ is C1-C6 alkyloxy;
Y is C1-C6 alkyloxy; and
X⁻ is an anion,
**characterized in that** an acridinium salt of the formula (III) : 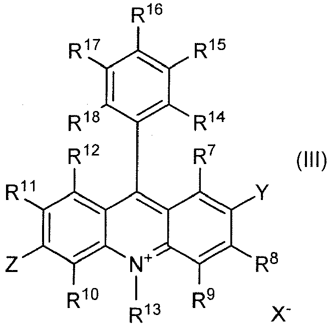
wherein Z is a leaving group, and the other symbols are as defined above,
is reacted with a nucleophile.

17. The process according to claim 16, wherein the nucleophile is a metal C1-C6 alkoxide or C1-C6 alkylamine.

18. The process according to claim 16 or 17, wherein the aclidinium salt of the formula (III) is that obtained by the process according to claim 14 or 15.
